(19) Europäisches Patentamt
European Patent Office
Office européen des brevets

(11) EP 1 503 756 B1

(12) EUROPEAN PATENT SPECIFICATION

(45) Date of publication and mention of the grant of the patent:
**12.08.2009 Bulletin 2009/33**

(21) Application number: **03725129.5**

(22) Date of filing: **30.04.2003**

(51) Int Cl.:
***A61K 31/425*** *(2006.01)* ***A61P 35/00*** *(2006.01)*

(86) International application number:
**PCT/EP2003/004581**

(87) International publication number:
**WO 2003/092683 (13.11.2003 Gazette 2003/46)**

(54) **EPOTHILONE DERIVATIVE FOR THE TREATMENT OF HEPATOMA AND OTHER CANCER DISEASES**

EPOTHILONDERIVAT ZUR BEHANDLUNG VON HEPATOMA UND ANDEREN KREBSERKRANKUNGEN

DES DERIVES D'EPOTHILONE POUR LE TRAITEMENT DE L'HEPATOME ET D'AUTRES MALADIES CANCEREUSES

(84) Designated Contracting States:
**AT BE BG CH CY CZ DE DK EE ES FI FR GB GR HU IE IT LI LU MC NL PT RO SE SI SK TR**

(30) Priority: **01.05.2002 US 377063 P**

(43) Date of publication of application:
**09.02.2005 Bulletin 2005/06**

(73) Proprietors:
- **NOVARTIS AG**
  **4056 Basel (CH)**
  Designated Contracting States:
  **BE BG CH CY CZ DE DK EE ES FI FR GB GR HU IE IT LI LU MC NL PT RO SE SI SK TR**
- **Novartis Pharma GmbH**
  **1230 Wien (AT)**
  Designated Contracting States:
  **AT**

(72) Inventor: **ROTHERMEL, John, David**
**Randolph, NJ 07869 (US)**

(74) Representative: **de Weerd, Petrus G.W. et al**
**Novartis International AG**
**Corporate Intellectual Property**
**4002 Basel (CH)**

(56) References cited:
**WO-A-01/10412** **US-B1- 6 302 838**

- **LEE F Y ET AL: "BMS-247550: a novel epothilone analog with a mode of action similar to paclitaxel but possessing superior antitumor efficacy." CLINICAL CANCER RESEARCH: AN OFFICIAL JOURNAL OF THE AMERICAN ASSOCIATION FOR CANCER RESEARCH. UNITED STATES MAY 2001, vol. 7, no. 5, May 2001 (2001-05), pages 1429-1437, XP002254263 ISSN: 1078-0432**
- **CHOU T C ET AL: "Desoxyepothilone B is curative against human tumor xenografts that are refractory to paclitaxel." PROCEEDINGS OF THE NATIONAL ACADEMY OF SCIENCES OF THE UNITED STATES OF AMERICA. UNITED STATES 22 DEC 1998, vol. 95, no. 26, 22 December 1998 (1998-12-22), pages 15798-15802, XP002254264 ISSN: 0027-8424**
- **ALTMANN K-H ET AL: "Epothilones and related structures - a new class of microtubule inhibitors with potent in vivo antitumor activity" BBA - REVIEWS ON CANCER, ELSEVIER SCIENCE BV, AMSTERDAM, NL, vol. 1470, no. 3, 17 May 2000 (2000-05-17), pages M79-M91, XP004281887 ISSN: 0304-419X**
- **"Phase I dose-escalating trial of KOS-862 (epothilone D) in patients with advanced malignancies" EUROPEAN JOURNAL OF CANCER, PERGAMON PRESS, OXFORD, GB, vol. 38, November 2002 (2002-11), page S41 XP004403564 ISSN: 0959-8049**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- STERNBERG C N: "What's new in the treatment of advanced prostate cancer?" EUROPEAN JOURNAL OF CANCER, PERGAMON PRESS, OXFORD, GB, vol. 39, no. 2, January 2003 (2003-01), pages 136-146, XP004400257 ISSN: 0959-8049
- O'CONNOR O A ET AL: "CLINICAL DEVELOPMENT OF THE NOVEL EPOTHILONE BMS-247550: FIRST OF A NEW CLASS OF TUBULIN POLYMERIZATION AGENTS WITH PROMISING ACTIVITY AGAINST CHEMOTHERAPY-REFRACTORY AGGRESSIVE AND MANTLE CELL LYMPHOMA" BLOOD, W.B.SAUNDERS COMPAGNY, ORLANDO, FL, US, vol. 98, no. 11, PART 1, 16 November 2001 (2001-11-16), pages 347A-348A, XP001120000 ISSN: 0006-4971
- WOLFF ET AL: "Epothilone A induces apoptosis in neuroblastoma cells with multiple mechanisms of drug resistance" INTERNATIONAL JOURNAL OF ONCOLOGY, EDITORIAL ACADEMY OF THE INTERNATIONAL JOURNAL OF ONCOLOGY,, GR, vol. 11, no. 1, July 1997 (1997-07), pages 123-126, XP002111280 ISSN: 1019-6439

## Description

The present invention relates to the use of an epothilone derivative of formula I as defined below for the preparation of a medicament for treating a warm-blooded animal, especially a human, having a cancer disease selected from hepatoma; primary fallopian tube cancer; primary peritoneal cancer; renal cell carcinoma progressing after treatment with a cytokine, radiotherapy and/or nephrectomy; ovarian cancer progressing after treatment with a platinum compound or radiotherapy; and metastasis thereof comprising administering to said animal a therapeutically effective amount of an epothilone derivative of formula I as defined below.

The epothilones, especially epothilones A, B and D, represent a new class of microtubule stabilizing cytotoxic agents (see Gerth, K. et al., J. Antibiot. 49, 560-3 (1996); or Hoefle et al., DE 41 38 042).

Surprisingly, now it was found that epothilone derivatives of formula I

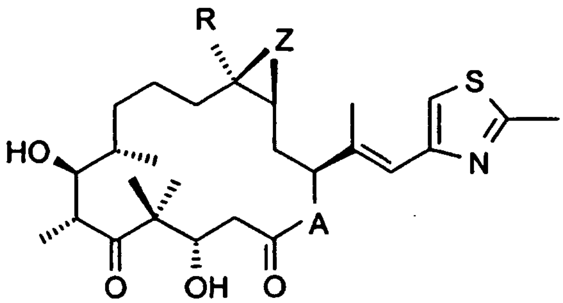

(I)

wherein A represents O or $NR_N$, wherein $R_N$ is hydrogen or lower alkyl, R is hydrogen or lower alkyl, and Z is O or a bond, produce a beneficial effect in the treatment of a cancer disease selected from hepatoma; primary fallopian tube cancer; primary peritoneal cancer; renal cell carcinoma progressing after treatment with a cytokine, radiotherapy and/or nephrectomy; ovarian cancer progressing after treatment with a platinum compound or radiotherapy; and metastasis thereof.

Hence, the invention relates to the use of a compound I for the manufacture of a medicament for treating a warm-blooded animal, preferably a human, having a cancer disease as mentioned above comprising administering a therapeutically effective amount of an epothilone derivative of formula I in which A represents O or $NR_N$, wherein $R_N$ is hydrogen or lower alkyl, R is hydrogen or lower alkyl, and Z is O or a bond, or a pharmaceutically acceptable salt thereof to a warm-blooded animal in need thereof.

The term "hepatoma" as used herein means a cancer disease characterized by at least one liver tumor arising from malignant hepatocytes. Hepatoma is an important cause of death in certain areas of Africa and Southeast Asia. Symptoms for the disease are abdominal pain, weight loss, a right upper quadrant mass and unexplained deterioration in a previously stable patient with cirrhosis. Sometimes systemic metabolic manifestation occurs, including hypoglycemia, erythrocytosis, hypercalcemia and hyperlipidemia. An elevated level of $\alpha$-fetoprotein is another indicator used in diagnosis. Abdominal US, CT and MRI are important diagnostic aids and can sometimes detect subclinical carcinomas. The treatment of hepatoma is generally unsatisfactory, since the tumor is not radiosensitive and chemotherapy is usually unsuccessful.

The term "primary fallopian tube cancer" as used herein includes, but is not limited to, a cancer disease that can be limited to one or both fallopian tubes with or without pelvic extension, but also a cancer involving peritoneal implants outside the pelvis and/or metastases.

The term "radiotherapy" as used herein includes, but is not limited to, the treatment of a disease by ionizing radiation.

The term "cytokine" as used herein includes, but is not limited to IL-2 and IFN-$\alpha$.

The term "orchiectomy" as used herein means the excision of one of both testes.

The term "platinum compound" as used herein includes, but is not limited to carboplatin, cisplatin and oxaliplatin.

The terms "treatment or "treating" as used herein comprises the treatment of patients having hepatoma, being in a pre-stage of said disease or were subject to a surgical resection of a liver tumor, which treatment effects a complete response, partial response or stable disease in said patients.

The term "complete response" as used herein means in particular to the resolution of all measurable or evaluable disease.

The term "partial response" as used herein means in particular a greater than or equal to 50 % reduction in measurable or evaluable disease in the absence of progression in any particular disease site.

The term "stable disease" as used herein means in particular a less than 50 % decrease or less than 25 % increase in measurable or evaluable disease.

The term "standard anti-diarrheal" as used herein include, but is not limited to, natural opiods, such as tincture of opium, paregoric, and codeine, synthetic opoids, such as diphenoxylate, difenoxin and loperamide, bismuth subsalicylate, octreotide, especially in the form as marketed under the tradename SANDOSTATIN LAR™, motilin antagonists and traditional antidiarrheal remedies, such as kaolin, pectin, berberine and muscarinic agents. The antidiarrheal agent is administered as a preventative measure throughout the treatment cycle or as needed when diarrhea occurs. The antidiarrheal agent is administered to prevent, control or eliminate diarrhea that is sometimes associated with the administration of epothilones, especially epothilone B.

Unless stated otherwise, in the present disclosure organic radicals and compounds designated "lower" contain not more than 7, preferably not more than 4, carbon atoms.

A compound of formula I in which A represents O, R is hydrogen and Z is O is known as epothilone A; a compound of formula I wherein A represents O, R is methyl and Z is O is known as epothilone B; a compound of formula I wherein A represents O, R is hydrogen and Z is a bond is known as epothilone C; a compound of formula I wherein A represents O, R is methyl and Z is a bond is known as epothilone D.

Epothilone derivatives of formula I in which A represents O or $NR_N$, wherein $R_N$ is hydrogen or lower alkyl, R is hydrogen or lower alkyl and Z is O or a bond, and methods for the preparation of such epothilone derivatives are in particular generically and specifically disclosed in the patents and patent applications WO 93/10121, US 6,194,181, WO 98/25929, WO 98/08849, WO 99/43653, WO 99/39694, WO 98/22461 and WO 00/31247 in each case in particular in the compound claims and the final products of the working examples. The subject-matter of the final products, the pharmaceutical preparations and the claims comprised by these patents and patent applications is hereby incorporated into the present application by reference to these publications. Comprised are likewise the corresponding stereoisomers as well as the corresponding crystal modifications, e.g. solvates and polymorphs, which are disclosed therein. The epothilone derivatives of formula I can be administered as described in the publications cited above, e.g., epothilone B, can be administered as part of pharmaceutical compositions which are disclosed in WO 99/39694, in particular epothilone B can be used formulated in polyethylene glycol 300 (PEG 300) which composition has to be pre-diluted in 0.9% sodium chloride solution to obtain a concentration of 1 mg/mL.

The transformation of epothilone B to the corresponding lactam is disclosed in Scheme 21 (page 31, 32) and Example 3 of WO 99/02514 (pages 48 - 50). The transformation of a compound of formula I which is different from epothilone B into the corresponding lactam can be accomplished analogously. Corresponding epothilone derivatives of formula I wherein $R_N$ is lower alkyl can be prepared by methods known in the art such as a reductive alkylation reaction starting from the epothilone derivative wherein $R_N$ is hydrogen.

It will be understood that in the discussion of methods, references to the active ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. The active ingredient or a pharmaceutically acceptable salt thereof may also be used in form of a hydrate or include other solvents used for crystallization.

The invention also provides the use of a compound of formula I for the preparation of a medicament for the treatment of a cancer disease selected from hepatoma; primary fallopian tube cancer; primary peritoneal cancer; breast cancer progressing after treatment with hormonal agents or radiotherapy; renal cell carcinoma progressing after treatment with a cytokine, radiotherapy and/or nephrectomy; melanoma progressing after radiotherapy; prostate cancer progressing after orchiectomy; ovarian cancer progressing after treatment with a platinum compound or radiotherapy; and colorectal cancer progressing after radiotherapy and/or treatment with oxaliplatin or irinotecan; and metastasis

In one preferred embodiment of the invention, an epothilone derivative of formula I is employed wherein A represents O, R is lower alkyl, especially methyl, ethyl or n-propyl, or hydrogen and Z is O or a bond. More preferably, an epothilone derivative of formula I is employed wherein A represents O, R is methyl and Z is O, which compound is also known as epothilone B.

In particular, the present invention relates to the use of a compound of formula I for the preparation of a medicament for treating a warm-blooded animal having hepatoma comprising administering a therapeutically effective amount of an epothilone derivative of formula I or a pharmaceutically acceptable salt thereof, especially hepatoma which is progressing after radiotherapy and/or cannot be controlled by surgical resection.

One embodiment of the invention pertains to the treatment of ovarian cancer progressing after treatment with a platinum compound or radiotherapy.

One embodiment of the invention pertains to the treatment of primary fallopian tube cancer, especially such cancer progressing after treatment with a platinum compound, a taxane or radiotherapy, preferably fallopian tube cancer which is a papillary serous adenocarcinoma.

One embodiment of the invention pertains to the treatment of primary peritoneal cancer progressing after

treatment with a platinum compound, a taxane or radiotherapy.

One embodiment of the invention pertains to the treatment of renal cell carcinoma progressing after treatment with a cytokine, radiotherapy and/or nephrectomy

The uses in treating a warm-blooded animal having a cancer disease as disclosed herein can be employed in the form of a monotherapy or in addition to other therapy forms, e.g., radiation or, in particular, together with the administration of a standard anti-diarrheal.

The structure of the active ingredients identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active ingredients and, based on these references, likewise enabled to manufacture and test the pharmaceutical indications and properties in standard test models, both *in vitro* and *in vivo.*

The person skilled in the pertinent art is fully enabled to select relevant test models to prove the herein before and hereinafter mentioned beneficial effects hepatoma of a compound of formula I.

The pharmacological activity of a compound of formula I, in particular epothilone B, can be demonstrated, e.g., in a study wherein patients suffering from hepatoma are treated with continuous 4-week cycles (three weeks on/one week off) of epothilone B until either disease progression or unacceptable side effects occur. Response initially can be evaluated after the first two cycles, and can be determined, e.g., by one of the diagnostic methods mentioned above and/or stabilization or improvement in clinical symptoms. Evaluations for response can be performed, e.g., every two cycles thereafter.

Preparations of a compound of formula I for enteral administration, such as nasal, buccal, rectal or, especially, oral administration, and for parenteral administration, such as intravenous, intramuscular or subcutaneous administration, to warm-blooded animals, especially humans, are especially preferred. The preparations contain the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

Preference is given to a pharmaceutical composition that is suitable for administration to a warm-blooded animal, especially a human or commercially useful mammal, suffering from a disease that is responsive to the inhibition of microtubule depolymerisation, for example psoriasis or especially a neoplastic disease, comprising a correspondingly effective amount of a compound of formula I, or a pharmaceutically acceptable salt thereof when salt-forming groups are present, together with at least one pharmaceutically acceptable carrier.

A pharmaceutical composition for the prophylactic or especially therapeutic treatment of neoplastic and other proliferative diseases of a warm-blooded animal, especially a human or a commercially useful mammal requiring such treatment, especially suffering from such a disease, comprising a new compound of formula I, or a pharmaceutically acceptable salt thereof, as active ingredient in a quantity that is prophylactically or especially therapeutically active against said diseases, is likewise preferred.

Pharmaceutical preparations contain from about 0.000001 % to 95 % of the active ingredient, whereby single-dose forms of administration preferably have from approximately 0.00001 % to 90 % and multiple-dose forms of administration preferably have from approximately 0.0001 to 0.5 % in the case of preparations for parenteral administration or 1 % to 20 % active ingredient in the case of preparations for enteral administration. Unit dose forms are, for example, coated and uncoated tablets, ampoules, vials, suppositories or capsules. Further dosage forms are, for example, ointments, creams, pastes, foams, tinctures, lipsticks, drops, sprays, dispersions, etc. Examples are capsules containing from about 0.0002 g to about 1.0 g active ingredient.

The pharmaceutical preparations of the present invention are prepared in a manner known *per se,* for example by means of conventional mixing, granulating, coating, dissolving or lyophilising processes.

Preference is given to the use of solutions of the active ingredient, and also suspensions or dispersions, especially isotonic aqueous solutions, dispersions or suspensions which, for example in the case of lyophilised preparations which contain the active ingredient on its own or together with a carrier, for example mannitol, can be made up before use. The pharmaceutical preparations may be sterilised and/or may contain excipients, for example preservatives, stabilisers, wetting agents and/or emulsifiers, solubilisers, salts for regulating the osmotic pressure and/or buffers and are prepared in a manner known *per se,* for example by means of conventional dissolving or lyophilising processes. The said solutions or suspensions may contain viscosity-increasing agents, typically sodium carboxymethylcellulose, carboxymethylcellulose, dextran, polyvinylpyrrolidone, or gelatin, or also solubilisers, for example ®Tween 80 [polyoxyethylene(20)sorbitan mono-oleate; trademark of ICI Americas, Inc, USA].

Suspensions in oil contain as the oil component the vegetable, synthetic, or semi-synthetic oils customary for injection purposes. In respect of such, special mention may be made of liquid fatty acid esters that contain as the acid component a long-chained fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brassidic acid or linoleic acid,

if desired with the addition of antioxidants, for example vitamin E, β-carotene or 3,5-di-tert-butyl-4-hydroxytoluene. The alcohol component of these fatty acid esters has a maximum of 6 carbon atoms and is a mono- or polyhydric, for example a mono-, di- or trihydric, alcohol, for example methanol, ethanol, propanol, butanol or pentanol or the isomers thereof, but especially glycol and glycerol. As fatty acid esters, therefore, the following are mentioned: ethyl oleate, isopropyl myristate, isopropyl palmitate, "Labrafil M 2375" (polyoxyethylene glycerol trioleate from Gattefossé, Paris), "Labrafil M 1944 CS" (unsaturated polyglycolised glycerides prepared by alcoholysis of apricot seed oil and consisting of glycerides and polyethylene glycol ester; Gattefossé, France), "Labrasol" (saturated polyglycolised glycerides prepared by alcoholysis of TCM and consisting of glycerides and polyethylene glycol ester; Gattefossé, France), and/or "Miglyol 812" (triglyceride of saturated fatty acids of chain length $C_8$ to $C_{12}$ from Hüls AG, Germany), but especially vegetable oils such as olive oil, cottonseed oil, almond oil, castor oil, sesame oil, soybean oil and more especially groundnut oil.

The manufacture of injectable preparations is usually carried out under sterile conditions, as is the filling, for example, into ampoules or vials, and the sealing of the containers.

Pharmaceutical compositions for oral administration can be obtained, for example, by combining the active ingredient with one or more solid carriers, if need be granulating a resulting mixture, and processing the mixture or granules, if desired, to form tablets or tablet cores, if need be by the inclusion of additional excipients.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores may be provided with suitable, if need be enteric, coatings, using *inter alia* concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

Orally administrable pharmaceutical compositions also include hard capsules consisting of gelatin, and also soft, sealed capsules consisting of gelatin and a plasticiser, such as glycerol or sorbitol. The hard capsules may contain the active ingredient in the form of granules, for example in admixture with fillers, such as corn starch, binders, and/or glidants, such as talc or magnesium stearate, and if need be stabilisers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilisers and detergents, for example of the polyoxyethylene sorbitan fatty acid ester type, may also be added.

Suitable rectally administrable pharmaceutical preparations are, for example, suppositories that consist of a combination of the active ingredient and a suppository base. Suitable suppository bases are, for example, natural or synthetic triglycerides, paraffin hydrocarbons, polyethylene glycols or higher alkanols.

The formulations suitable for parenteral administration are primarily aqueous solutions ([or example in physiological saline, obtainable by diluting solutions in polyethylene glycol, such as polyethylene glycol (PEG) 300 or PEG 400] of an active ingredient in water-soluble form, e.g. a water-soluble salt, or aqueous injectable suspensions containing viscosity-increasing agents, e.g. sodium carboxymethyl cellulose, sorbitol and/or dextran, and where appropriate stabilisers. The active ingredient, if need be together with excipients, can also be in the form of a lyophilisate and can be made into a solution before parenteral administration by the addition of suitable solvents.

Solutions such as those used, for example, for parenteral administration can also be employed as infusion solutions.

Preferred preservatives are, for example, antioxidants, such as ascorbic acid, or microbicides, such as sorbic acid or benzoic acid.

The effective dosage of a compound of formula I may vary depending on the particular compound or pharmaceutical composition employed, the mode of administration, the severity of hepatoma being treated. Thus, the dosage regimen is selected in accordance with a variety of factors including the route of administration and the renal and hepatic function of the patient. A physician, clinician or veterinarian of ordinary skill can readily determine and prescribe the effective amount of a compound of formula I required to prevent, counter or arrest the progress of the condition. Optimal precision in achieving concentration of the active ingredients within the range that yields efficacy without toxicity requires a regimen based on the kinetics of the active ingredients' availability to target sites.

If the warm-blooded animal is a human, the dosage of a compound of formula I is preferably in the range of about 0.1 to 75 mg/m, preferably 0.25 to 50 mg/m, e.g. 2.5 or 6 mg/$m^2$, once weekly for two to four, e.g. three, weeks,

followed by 6 to 8 days off in the case of an adult patient.

Epothilone B is preferably administered in a dose that allows for the treatment of the cancer diseases mentioned herein and which dose is calculated according to the formula (I)

$$\text{single dose (mg/m2)} = (0.1 \text{ to } y) \times N \qquad \text{(I)}$$

wherein N is the number of weeks between treatments and y is 6, wherein epothilone B is administered in more than one treatment cycle after an interval of one week to six weeks after the preceding treatment.

In one embodiment of the invention, epothilone B is administered weekly in a dose that is between about 0.1 to 6 mg/$m^2$, preferably between 0.1 and 3 mg/$m^2$, e.g. 2.5 or 3.0 mg/$m^2$, for three weeks after an interval of one to six weeks, especially an interval of one week, after the preceding treatment. In another embodiment of the invention said epothilone B is preferably administered to a human every 18 to 24 days in a dose that is between about 0.3 and 12 mg/$m^2$.

The present invention also provides the use of a compound of formula I as defined herein for the treatment of a cancer disease as mentioned herein and for the preparation of a medicament for the treatment of such a cancer disease.

Moreover, the present invention provides a commercial package comprising as active ingredients a compound of formula I together with instructions for use thereof in the treatment of a cancer disease.

## Examples

### Example 1

An open-label, single-arm, two-stage, multi-center study investigating the efficacy of 7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione administered intravenously at 2.5 mg/m2 as a 5 minute bolus infusion repeated every week for three weeks followed by one week off in patients who have ovarian, primary Fallopian, or primary peritoneal cancer. Patients will have either failed to respond to prior first-line taxane/platinum therapy, or have relapsed within six months of completing such therapy. Chemotherapies other than taxane/platinum or an additional agent to this regimen will not be allowed. The study population for this trial consists of ovarian, primary Fallopian, or primary peritoneal cancer patients who have either failed to respond to first-line therapy of taxane/platinum (or a combination using these agents), or patients who initially responded but have relapsed within six months of completing therapy. A third therapeutic agent administered as part of the taxane/platinum therapy is permitted for refractory disease if all other criteria are met.

This study is on going. Several patients show a positive response in this study.

### Example 2

An open-label, single-arm, two-stage multicenter study investigating the efficacy of 7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione administered intravenously at 2.5 mg/m2 as a 5 minute bolus infusion repeated every week for three weeks followed by one week off in patients with advanced renal cancer. 7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione is administered once every week for three weeks followed by a one-week drug holiday until either progression of disease or unacceptable toxicities occurs. Each four-week period is considered one cycle. Patients are evaluated for tumor response one week after their last dose of every other cycle, starting with Cycle 2. Patients may, at any time, have their dose reduced to 2.0 mg/m2 if in the investigator's opinion the original dose is either intolerable or unsafe for any given patient, and without such a dose reduction the patient would be forced to withdraw from the study. All dose modifications are captured on the dose administration record CRF. The study period for efficacy evaluation is up to six cycles. The study will enroll patients with histologically confirmed transitional cell carcinoma of the kidney. Mixed histology with a transitional cell carcinoma component is allowed. Patients may have either progressive regional disease or metastatic disease.

This trial is currently ongoing. Several patients show a positive response in this study.

### Example 3

An open-label, single-arm, two-stage multicenter study to investigate the efficacy of 7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione administered intravenously at 2.5 mg/m2 as a 5 minute bolus infusion repeated every week for three weeks followed by one week off in patients with advanced colorectal cancer. 7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione is administered once every week for three weeks followed by a one-week drug holiday until either progression of disease or unacceptable toxicities occurs. Each four-week period is considered one cycle. Patients are evaluated for tumor response one week after their last dose of every other cycle, starting with Cycle 2. Patients may, at any time, have their dose reduced to 2.0 mg/$m^2$ if in the investigator's opinion the original dose is either intolerable or unsafe for any given patient, and without such a dose reduction the patient will be forced to withdraw from the study. All dose modifications are captured on the dose administration record CRF. The study period for efficacy evaluation is up to six cycles. The study population for this trial consists of colorectal cancer patients who have failed prior chemotherapy containing fluoropyrimidine (such as 5-FU and Xeloda) and either Irinotecan or oxaliplatin either administered in combination or sequentially (one regimen containing 5-flurouracil and one subsequent regimen containing either Irinotecan or oxaliplatin). The prior chemotherapy may have been administered either as adjuvant therapy or as treatment for metastatic disease. Patients who have received only adjuvant therapy for their disease are eligible, as long as they have relapsed within six months of completing such therapy, and that therapy contained 5-FU and Irinotecan or oxaliplatin administered in combination as part of an investigational protocol. This trial is ongoing. Several patients shown a positive response in this trial.

Example 4

An open-label, single-arm, two-stage, multicenter study to investigate the efficacy of 7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione administered intravenously at 6.0 mg/$m^2$ as a 5 minute bolus infusion repeated once every three weeks in patients with advanced colorectal cancer. 7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione is administered once every three weeks until either progression of disease or unacceptable toxicities occurs. Each three-week period is considered one cycle. Patients are evaluated for tumor response one week after the last dose of every third cycle, starting with Cycle 3. Patients may, at any time, have their dose reduced to 5.4 mg/$m^2$ or 4.8 mg/$m^2$ if in the investigator's opinion the original dose is either intolerable or unsafe for any given patient, and without such a dose reduction the patient will be forced to withdraw from the study. All dose modifications are captured on the Dose Administration Record CRF.
The study period for efficacy evaluation is up to nine cycles.
The study population for this trial consists of colorectal cancer patients who have failed prior chemotherapy containing fluoropyrimidine (such as 5-FU and Xeloda) and either Irinotecan or oxaliplatin either administered in combination or sequentially (one regimen containing 5-flurouracil and one subsequent regimen containing either Irinotecan or oxaliplatin). The prior chemotherapy may have been administered either as adjuvant therapy or as treatment for metastatic disease. Patients who have received only adjuvant therapy for their disease are eligible, as long as they have relapsed within six months of completing such therapy and that therapy contained 5-FU and Irinotecan or oxaliplatin administered in combination as part of an investigational protocol.
This trial is ongoing. Several patients shown a positive response in this trial.

Example 5

Open-label, single-arm, two-stage, multicenter study to investigate the efficacy of EP0906 administered intravenously at 2.5 mg/$m^2$ as a 5 minute bolus infusion once a week for three weeks followed by one week off in patients with androgen-independent prostate cancer who are chemo-naïve or who have failed one prior chemotherapy. 7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione is administered once every week for three weeks followed by a one-week drug holiday until either progression of disease or unacceptable toxicities occurs. Each four-week period is considered one cycle. Patients are evaluated for tumor response one week after their last dose of every other cycle, starting with Cycle 2. Patients may, at any time, have their dose reduced to 2.0 mg/$m^2$ if in the investigator's opinion the original dose is either intolerable or unsafe for any given patient, and without such a dose reduction the patient will be forced to withdraw from the study. All dose modifications are captured on the dose administration record CRF. The study period for efficacy evaluation is up to six cycles.
The study population for this trial consists of androgen-independent prostate cancer patients who have demonstrated evidence of progressive disease.

- Patients with any histologically proven prostate cancer with measurable metastatic disease or PSA progression > 20ng/ml after initial hormonal therapy will be eligible.
- Patients must be maintained on androgen ablation therapy with a LHRH agonist or have undergone orchiectomy.
- Patients in whom bicalutamide or flutamide has been recently withdrawn must demonstrate progression of disease and be at least 6 weeks and 4 weeks respectively, beyond the discontinuation of such agents. Patients taking PC-SPES must discontinue therapy for a minimum of 4 weeks.
- For patients with disease progression defined solely by PSA increase: two consecutive rises in PSA measurement, over a 4-week period (each separated from the previous by 2 weeks). The last measurement must be at least 50% greater than the nadir PSA achieved after the last therapeutic maneuver. For patients who discontinued bicalutamide therapy prior to study entry, a third rising PSA measurement is required 2 weeks from the second PSA measurement (i.e. over a 6 week period).

This trial is ongoing. Several patients show a positive response in this trial.

Example 6: Soft capsules

5000 soft gelatin capsules, each containing as active ingredient 0.05 g of one of the compounds of formula I are prepared as follows:

| composition | |
|---|---|
| active ingredient | 250 g |
| Lauroglycol | 2 litres |

Preparation process: The pulverised active ingredient is suspended in Lauroglykol® (propylene glycol laurate, Gattefossé S.A., Saint Priest, France) and ground in a wet pulverizer to a grain size of approximately 1 to 3 μm. Portions each containing 0.419 g of the mixture are then filled into soft gelatin capsules by a capsule filling machine.

Example 7: Infusion solution

The compound of example 1, 2, 3 or 4 is dissolved at a concentration of 1 mg/ml in polyethylene glycol 300 (PEG 300) and filled into 2 ml vials. For infusion, this solution is diluted with 50 to 100 ml of 0.9% saline according to US Pharmacopoeia.

## Claims

1. The use of a compound of formula I

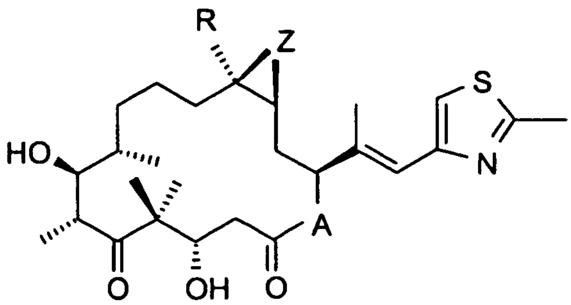

(I)

wherein A represents O or $NR_N$, wherein $R_N$ is hydrogen or lower alkyl, R is hydrogen or lower alkyl, and Z is O or a bond, or a pharmaceutically acceptable salt thereof in need thereof;
for the preparation of a medicament for the treatment of a cancer disease selected from hepatoma; primary fallopian tube cancer; primary peritoneal cancer; renal cell carcinoma progressing after treatment with a cytokine, radiotherapy and/or nephrectomy; ovarian cancer progressing after treatment with a platinum compound or radiotherapy; and metastasis thereof.

2. The use according to claim 1 in which an epothilone derivative of formula I wherein A represents O, R is methyl and Z is O or a pharmaceutically acceptable salt thereof is administered.

3. The use according to claim 2 wherin the epothilone derivative is in a dose that allows for the treatment of said disease and which dose is calculated according to the formula (I)

$$\text{single dose (mg/m2)} = (0.1 \text{ to } y) \times N \qquad \text{(I)}$$

wherein N is the number of weeks between treatments and y is 6, wherein said epothilone is administered in more than one treatment cycle after an interval of one week to six weeks after the preceding treatment.

4. The use according to claim 3 comprising administering said epothilone derivative weekly in a dose that is between about 0.1 to 6 $mg/m^2$.

5. The use according to claim 3 comprising administering said epothilone derivative weekly in a dose that is between about 0.1 to 6 $mg/m^2$ for three weeks after an interval of one to six weeks after the preceding treatment.

6. The use according to claim 3 comprising administering said epothilone derivative every third week in a dose that is between about 0.3 to 12 $mg/m^2$.

7. The use according to claim 1 wherein the cancer disease is hepatoma.

8. The use according to claim 7 wherein the cancer disease is hepatoma progressing after radiotherapy.

9. The use according to claim 1 wherein the cancer disease is ovarian cancer progressing after treatment with a platinum compound or radiotherapy.

10. The use according to claim 1 wherein the cancer disease is primary fallopian tube cancer.

11. The use according to claim 1 wherein the cancer disease is primary fallopian tube cancer progressing after treatment with a platinum compound, a taxane or radiotherapy.

12. The use according to claim 10 or 11 wherein the fallopian tube cancer is papillary serous adenocarcinoma.

13. The use according to claim 1 wherein the cancer disease is primary peritoneal cancer progressing after treatment with a platinum compound, a taxane or radiotherapy.

14. The use according to claim 1 wherein the cancer disease is renal cell carcinoma progressing after treatment with a cytokine, radiotherapy and/or nephrectomy.

**Patentansprüche**

1. Verwendung einer Verbindung der Formel I

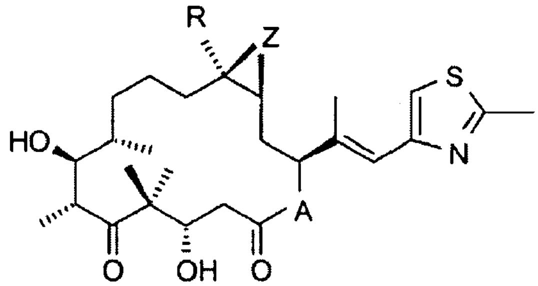

(I)

worin A für O oder $NR_N$ steht, worin $R_N$ für Wasserstoff oder Niederalkyl steht, R für Wasserstoff oder Niederalkyl steht und Z für O oder eine Bindung steht, oder bedarfsweise eines pharmazeutisch akzeptablen Salzes hiervon, zur Herstellung eines Arzneimittels für die Behandlung einer Krebskrankheit, die ausgewählt ist aus Hämatom, primärem Eileiterkrebs, primärem Peritonealkrebs, Nierenzellkarzinom, das nach einer Behandlung mit einem Cytokin, einer Radiotherapie und/oder einer Nephrektomie fortschreitet, Ovarialkarzinom, das nach einer Behandlung mit einer Platinverbindung oder einer Radiotherapie fortschreitet, und Metastasen hiervon.

**2.** Verwendung nach Anspruch 1, worin ein Epothilonderivat der Formel I, worin A für O steht, R für Methyl steht und Z für O steht, oder ein pharmazeutisch akzeptables Salz hiervon verabreicht wird.

**3.** Verwendung nach Anspruch 2, worin das Epothilonderivat in einer Dosis vorliegt, die eine Behandlung einer der oben erwähnten Krankheiten erlaubt und die nach der folgenden Formel (I) berechnet ist

$$\text{Einzeldosis (mg/m}^2\text{)} = (0{,}1 \text{ bis } y) \times N \qquad \textbf{(I)}$$

worin N die Anzahl an Wochen zwischen Behandlungen ist und y für 6 steht, worin die Verabreichung des Epothilons in mehr als einem Behandlungszyklus nach einem Intervall von 1 bis 6 Wochen nach der vorhergehenden Behandlung erfolgt.

**4.** Verwendung nach Anspruch 3, worin die Verabreichung des Epothilonderivats in einer wöchentlichen Dosis zwischen etwa 0,1 bis 6 mg/$m^2$ erfolgt.

**5.** Verwendung nach Anspruch 3, worin die Verabreichung des Epothilonderivats in einer wöchentlichen Dosis zwischen etwa 0,1 und 6 mg/$m^2$ während 3 Wochen nach einem Intervall von 1 bis 6 Wochen nach der vorhergehenden Behandlung erfolgt.

**6.** Verwendung nach Anspruch 3, worin die Verabreichung des Epothilonderivats jede dritte Woche in einer Dosis zwischen etwa 0,3 bis 12 mg/$m^2$ erfolgt.

**7.** Verwendung nach Anspruch 1, worin die Krebskrankheit Hepatom ist.

**8.** Verwendung nach Anspruch 7, worin die Krebskrankheit Hepatom ist, das nach einer Radiotherapie fortschreitet.

**9.** Verwendung nach Anspruch 1, worin die Krebskrankheit ein Ovarialkarzinom ist, das nach einer Behandlung mit einer Platinverbindung oder einer Radiotherapie fortschreitet.

**10.** Verwendung nach Anspruch 1, worin die Krebskrankheit ein primärer Eileiterkrebs ist.

**11.** Verwendung nach Anspruch 1, worin die Krebskrankheit ein primärer Eileiterkrebs ist, der nach einer Behandlung mit einer Platinverbindung, einem Taxan oder einer Radiotherapie fortschreitet.

**12.** Verwendung nach Anspruch 10 oder 11, worin der Eieleiterkrebs ein Papilla-seröses Adenokarzinom ist.

**13.** Verwendung nach Anspruch 1, worin die Krebskrankheit ein primärer Peritonealkrebs ist, der nach einer Behandlung mit einer Platinverbindung, einem Taxan oder einer Radiotherapie fortschreitet.

**14.** Verwendung nach Anspruch 1, worin die Krebskrankheit ein Nierenzellkarzinom ist, das nach einer Behandlung mit einem Cytokin, einer Radiotherapie und/oder einer Nephrektomie fortschreitet.

## Revendications

**1.** Utilisation d’un composé de formule 1

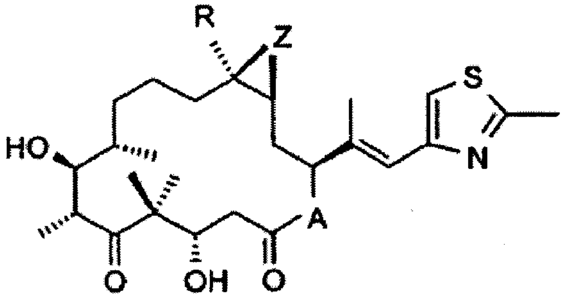

(I)

dans laquelle A représente O ou $NR_N$, où $R_N$ représente l'hydrogène ou un alkyle inférieur, R représente l'hydrogène ou un alkyle inférieur et Z représente O ou une liaison, ou de l'un de ses sels acceptables sur le plan pharmaceutique, pour la préparation d'un médicament destiné au traitement d'une maladie du type cancéreux, sélectionnée parmi l'hépatome, le cancer primitif de la trompe de Fallope, le cancer primitif péritonéal, le carcinome à cellules rénales progressant consécutivement à un traitement par une cytokine, une radiothérapie et/ou une néphrectomie, le cancer de l'ovaire progressant consécutivement à un traitement par un composé du platine ou une radiothérapie, et leurs métastases.

2. Utilisation selon la revendication 1, dans laquelle on administre un dérivé d'épothilone de formule I dans laquelle A représente O, R représente un méthyle et Z représente O, ou l'un de ses sels pharmaceutiquement acceptables.

3. Utilisation selon la revendication 2, dans laquelle le dérivé d'épothilone est dosé de façon à permettre le traitement de ladite maladie, la dose étant calculée selon la formule (I) suivante:

$$\text{dose unique (mg/m}^2\text{)} = (0{,}1 \text{ à } y) \times N \qquad \text{(I)}$$

dans laquelle N correspond au nombre de semaines entre les traitements et y vaut 6, ladite épothilone étant administrée sur plus d'un cycle de traitement après un intervalle d'une à six semaine(s) suivant le traitement précédent.

4. Utilisation selon la revendication 3, comprenant l'administration hebdomadaire dudit dérivé d'épothilone à une dose comprise entre environ 0,1 et 6 $mg/m^2$.

5. Utilisation selon la revendication 3, comprenant l'administration hebdomadaire dudit dérivé d'épothilone à une dose comprise entre environ 0,1 et 6 $mg/m^2$ pendant trois semaines après un intervalle d'une à six semaines après le traitement précédent.

6. Utilisation selon la revendication 3, comprenant l'administration dudit dérivé d'épothilone toutes les trois semaines à une dose comprise entre environ 0,3 et 12 $mg/m^2$.

7. Utilisation selon la revendication 1, ladite maladie du type cancéreux étant un hépatome.

8. Utilisation selon la revendication 7, ladite maladie du type cancéreux étant un hépatome progressant consécutivement à une radiothérapie.

9. Utilisation selon la revendication 1, ladite maladie du type cancéreux étant un cancer de l'ovaire progressant consécutivement à un traitement par un composé du platine ou une radiothérapie.

10. Utilisation selon la revendication 1, ladite maladie du type cancéreux étant un cancer primitif de la trompe de Fallope.

11. Utilisation selon la revendication 1, ladite maladie du type cancéreux étant un cancer primitif de la trompe de Fallope progressant consécutivement à un traitement par un composé du platine, un taxane ou une radiothérapie.

12. Utilisation selon la revendication 10 ou 11, ledit cancer de la trompe de Fallope étant un adénocarcinome papillaire séreux.

13. Utilisation selon la revendication 1, ladite maladie du type cancéreux étant un cancer primitif péritonéal progressant consécutivement à un traitement par un composé du platine, un taxane ou une radiothérapie.

14. Utilisation selon la revendication 1, ladite maladie du type cancéreux étant un carcinome à cellules rénales progressant consécutivement à un traitement par une cytokine, une radiothérapie et/ou une néphrectomie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 4138042
- WO 9310121 A
- US 6194181 B
- WO 9825929 A
- WO 9808849 A
- WO 9943653 A
- WO 9939694 A
- WO 9822461 A
- WO 0031247 A
- WO 9902514 A


### Non-patent literature cited in the description


- **Gerth, K. et al.** *J. Antibiot.,* 1996, vol. 49, 560-3